# FASCICULE DE BREVET EUROPEEN

(11) **EP 1 112 064 B1**
(45) Date de publication et mention de la délivrance du brevet: **15.01.2003**
(21) Numéro de dépôt: 99941732.2
(22) Date de dépôt: 10.09.1999
(51) Int. Cl.: A61K 9/16, A61K 9/48, A61K 31/215

(54) **PROCEDE DE PREPARATION DE NOUVELLES FORMULATIONS GALENIQUES DU FENOFIBRATE, FORMULATIONS GALENIQUES OBTENUES PAR LEDIT PROCEDE ET LEURS APPLICATIONS**
VERFAHREN ZUR HERSTELLUNG NEUER FENOFIBRATHALTIGER ARZNEIFORMULIERUNGEN, DADURCH HERGESTELLTE ARZNEIFORMULIERUNGEN UND IHRE VERWENDUNG
METHOD FOR PREPARING NOVEL FENOFIBRATE GALENIC FORMULATIONS, GALENIC FORMULATIONS OBTAINED AND APPLICATIONS

(30) Priorité: 17.09.1998 FR 9811611
(43) Date de publication de la demande: 04.07.2001
(73) Titulaire: CLL Pharma, Arenas, 06200 Nice (FR)
(72) Inventeur: LARUELLE, Claude, F-06270 Villeneuve-Loubet (FR); GIMET, René, F-06560 Valbonne (FR); TOSELLI, Dominique, F-06000 Nice (FR)
(74) Mandataire: Goulard, Sophie
(86) Numéro de dépôt international: FR9902155
(87) Numéro de publication internationale: WO00016749

(56) Documents cités:
- EP-A- 0 330 532
- EP-A- 0 793 958
- WO-A-96/36318
- WO-A-98/31361
- FR-A- 2 722 984

## Description

La présente Invention se rapporte à un procédé permettant de préparer des formulations galéniques comprenant du fénofibrate en tant que principe actif, et permettant de conférer à ce principe actif, lorsqu'il est absorbé oralement, une biodisponibilité significativement améliorée (*"suprabiodisponibilité"*) par rapport à celle obtenue avec les compositions pharmaceutiques à base de fénofibrate actuellement disponibles, aux formulations galéniques obtenues par ledit procédé ainsi qu'aux applications de ce procédé et de ces formulations galéniques, notamment pour la préparation de médicaments destinés à une administration par voie orale.

Le fénofibrate (DCI) ou 2-(4-(4-chlorobenzoyl)-phénoxy)-2-méthyl propionate d'isopropyle est un des hypolipidémiants les plus utilisés dans le monde dans le traitement des hypercholestérolémies et des hypertriglycéridémies endogènes de l'adulte isolées ou associées. Son efficacité dans ces indications thérapeutiques a été largement démontrée. Ainsi, administré au long cours à des doses thérapeutiquement efficaces, le fénofibrate permet d'abaisser la cholestérolémie de 20 à 25% et la triglycéridémie de 40 à 50% et ce, dès le premier mois de traitement.

Le fénofibrate a été mis sur le marché pour la première fois en France en 1975, sous la forme de gélules dosées à 100 mg de principe actif (LIPANTHYL® 100) et avec une posologie de 3 ou 4 gélules par jour à répartir entre les différents repas, correspondant donc à une administration quotidienne de 300 à 400 mg de principe actif. Cette spécialité pharmaceutique est toujours commercialisée à ce jour en France, mais sous le nom SECALIP® 100.

En 1986, un deuxième dosage destiné à offrir aux patients ne nécessitant que 300 mg de fénofibrate par jour une meilleure observance thérapeutique, a été commercialisé sous la forme de gélules renfermant 300 mg de principe actif (LIPANTHYL® 300), la posologie étant alors d'une seule gélule par jour. Une telle posologie est rendue possible par la longue durée d'action du fénofibrate dont la demi-vie d'élimination est, en effet, d'environ 20 heures. Cette spécialité pharmaceutique est également toujours disponible en France, mais sous le nom SECALIP® 300.

Depuis les travaux de DESAGER et al. (*J. Clin. pharmacol*., 1978, 26, 570-574) et ceux de WEIL et al. (*Drug metabolism*, 1980, 18, 115-120), on sait que, lorsque le fénofibrate est administré sous une forme solide (poudre ou granules), sa résorption intestinale est très incomplète puisque l'acide fénofibrique qui est son principal métabolite actif circulant, ne se retrouve qu'à hauteur de 20% de la dose de fénofibrate administrée si l'administration est faite à jeun, et à hauteur de 60% si l'administration est faite au cours d'un repas. Cette dernière donnée a été confirmée ultérieurement par STROLIN BENEDETTI et ses collaborateurs (*Acta Pharmacol.Toxicol*., 1986, 59, suppl. 5, 167) qui ont montré qu'après administration d'une gélule dosée à 300 mg de fénofibrate au cours d'un repas, seuls 60% de la dose de fénofibrate administrée sont effectivement résorbés et atteignent la circulation sanguine sous la forme d'acide fénofibrique.

Il s'avère, par ailleurs, qu'un traitement au long cours par le fénofibrate n'est pas totalement dénué d'effets indésirables, des cas d'atteintes musculaires (myalgies diffuses, sensibilité douloureuse, rhabdomyolyse, ...), de troubles digestifs, d'élévation des transaminases et de lithiases biliaires s'opposant au maintien d'un tel traitement ayant, en effet, été rapportés.

De ce fait, un certain nombre de travaux ont été réalisés dans le but de développer des formulations galéniques propres à augmenter la biodisponibilité du fénofibrate lorsque celui-ci est absorbé par voie orale, de manière à diminuer la dose de fénofibrate nécessaire à l'obtention d'un bénéfice thérapeutique et à diminuer, par voie de conséquence, les risques d'effets indésirables.

La Demande de Brevet Français n° 80 24568, déposée en 1980 aux noms conjoints de Claude LARUELLE et de DESHORS, représente une première avancée technologique en ce sens. En effet, ce Brevet décrit une formulation galénique se présentant sous la forme de granules qui comprennent un noyau neutre recouvert d'une couche intermédiaire à base de fénofibrate et d'une couche externe de protection formée d'un polymère compatible avec une administration orale et qui, réunis en gélules dosées à 250 mg, permettent d'obtenir, par une seule prise quotidienne, des taux plasmatiques d'acide fénofibrique thérapeutiquement efficaces.

La Demande de Brevet Européen n° 0 256 933, déposée en 1987 au nom d'ETHYPHARM, décrit une formulation galénique propre à favoriser notamment la résorption intestinale du fénofibrate et qui se présente également sous la forme de granules constitués par un noyau neutre, une couche intermédiaire à base de fénofibrate et une couche externe de protection, mais ces granules ont pour particularité, que le fénofibrate y est présent sous la forme de particules cristallines de dimension inférieure ou égale à 50 µm et, de préférence, de l'ordre de 10 µm.

La Demande de Brevet Français n° 88 02359, déposée en 1988 au nom de FOURNIER INNOVATION ET SYNERGIE, propose, elle, une composition thérapeutique se présentant sous la forme de gélules dosées à 200 mg de fénofibrate et comprenant du fénofibrate et un agent tensioactif solide ayant été soumis à une comicronisation. Cette composition thérapeutique, qui est commercialisée en France depuis 1991 sous le nom de LIPANTHYL® 200 Micronisé, représente incontestablement un progrès puisque des études ont mis en évidence qu'il existe une bioéquivalence cinétique entre une gélule de cette composition et une gélule de LIPANTHYL® 300 (GUICHARD et LEVY-PRADES SAURON, *J. Int. Med.,* 1991, 48-50), ainsi qu'entre une gélule de LIPANTHYL® 200 Micronisé et 3 gélules de LIPANTHYL® 100 (Laboratoires FOURNIER, *LIPANTHYL, Documentation scientifique et technique destinée aux Pharmaciens des Hôpitaux et des Etablissements de soins,* 1992).

Par ailleurs, BRODIE et ses collaborateurs *(Arzneimittel Forschung,* 1976, 26, 896-901) ont montré que l'administration du fénofibrate, qui est une molécule lipophile, dans de l'huile de tournesol permet d'obtenir une résorption intestinale presque complète de ce principe actif contrairement à son administration sous forme solide. Toutefois, il s'avère que l'utilisation de l'huile de tournesol comme solvant du fénofibrate ne peut pas être envisagée pour la réalisation de médicaments, en raison de ce qu'elle requiert un volume relativement important d'huile, de l'ordre de 5 ml, incompatible avec une mise en forme dans des capsules de volume convenable et acceptable par les patients.

Aussi, il a été proposé dans la Demande de Brevet Internationale WO 96/21439, déposée en 1996 au nom de GALEPHAR P.R., de réaliser un médicament sous la forme de gélules remplies d'un mélange constitué par du fénofibrate dissous dans des glycérides polyglycosylés comme, par exemple, des mélanges d'esters de glycérol et d'acides gras ou d'esters de polyéthylène glycols et d'acides gras. Selon cette Demande, 3 gélules dosées à 67 mg de fénofibrate d'un tel médicament seraient bioéquivalentes à 3 gélules de LIPANTHYL® 100, tandis qu'une gélule dosée à 200 mg de ce même médicament serait bioéquivalente à une gélule de LIPANTHYL® 200 Micronisé.

Parallèlement, dans la Demande de Brevet Français n° 95 09142, déposée en 1995 au nom de CL PHARMA, il a été suggéré d'améliorer la dissolution du fénofibrate et, partant, sa biodisponibilité en l'administrant sous la forme de capsules molles dans lesquelles il se trouve en solution dans un agent surfactif non ionique et amphiphile, à savoir l'éther monoéthylique du diéthylène glycol. Cette formulation galénique permet d'obtenir, par une administration quotidienne de 100 mg de fénofibrate, des concentrations plasmatiques en acide fénofibrique considérées comme thérapeutiquement efficaces. Toutefois, le coût de fabrication de telles capsules limite l'intérêt de leur exploitation.

Or, les Inventeurs, dans le cadre de leurs recherches sur l'amélioration de la biodisponibilité du fénofibrate, ont constaté que, de manière surprenante, il est possible d'obtenir des formulations galéniques qui, tout en se présentant sous une forme solide, permettent de conférer au fénofibrate, lorsqu'il est administré par voie orale, une biodisponibilité significativement plus élevée que celle obtenue avec les compositions pharmaceutiques à base de fénofibrate actuellement disponibles et d'offrir, ainsi, un bénéfice thérapeutique identique pour des doses de ce principe actif notablement plus faibles, en soumettant du fénofibrate micronisé à une granulation en présence d'un milieu liquide comprenant à la fois de l'eau, un alcool hydromiscible et un agent tensioactif, et en séchant le granulat ainsi préparé.

La présente Invention a, donc, pour objet un procédé de préparation d'une formulation galénique comprenant du fénofibrate en tant que principe actif, caractérisé en ce qu'il comprend :
(a) la micronisation du fénofibrate,
(b) la granulation du fénofibrate ainsi micronisé en présence d'un milieu liquide comprenant un agent tensioactif, de l'eau et un alcool hydromiscible, et
(c) le séchage du granulat ainsi obtenu.

Selon une première disposition avantageuse de ce procédé, il comprend de plus, préalablement et/ou postérieurement à l'opération de granulation, l'addition d'un ou plusieurs excipients choisis parmi les agents liants, les agents diluants, les agents désintégrants, les agents lubrifiants, les agents d'écoulement, les colorants et les agents de sapidité classiquement utilisés pour la préparation de formulations galéniques solides.

Ainsi, par exemple, le fénofibrate micronisé pourra être avantageusement mélangé, préalablement à la granulation, à un ou plusieurs agents liants tels que l'amidon, l'amidon prégélatinisé, les sucres (lactose, glucose, dextrose, ...), la polyvinylpyrrolidone, la méthylcellulose, l'éthylcellulose, la carboxyméthylcellulose ou la cellulose microcristalline, et/ou à un ou plusieurs agents diluants tels que le lactose, le kaolin, le mannitol, l'amidon, le chlorure de sodium ou le phosphate de calcium, et/ou encore à un ou plusieurs agents désintégrants du type amidon, carboxyméthyl amidon sodique, craie, carboxyméthylcellulose ou acide alginique, tandis qu'il pourra être avantageux d'ajouter au granulat résultant de la granulation, une fois sec, un ou plusieurs agents lubrifiants tels que le stéarate de magnésium, le stéarate de calcium ou le talc, les amidons, la cellulose et ses dérivés et/ou un ou plusieurs agents d'écoulement du type silice colloïdale, stéarate de magnésium, carboxyméthylamidon sodique, phosphate dicalcique, mannitol granulé, cellulose microcristalline, et/ou un ou plusieurs agents colorants et/ou encore un ou plusieurs agents de sapidité, selon la constitution que l'on souhaite donner à la formulation galénique et sa destination (mise en gélules, compression pour l'obtention de comprimés, ...).

Selon une autre disposition avantageuse du procédé conforme à l'Invention, il comprend en outre, postérieurement au séchage du granulat et antérieurement à son mélange éventuel avec un ou des excipients, le calibrage de ce granulat, lequel est avantageusement conduit dans des conditions permettant d'éliminer tous les grains présentant une taille supérieure ou égale à 150 µm.

Selon encore une autre disposition avantageuse de ce procédé, la micronisation du fénofibrate est réalisée de manière à obtenir une poudre dont les particules ont une dimension homogène égale au plus à 10 µm et, de préférence, comprise entre 5 et 10 µm.

Selon une disposition préférée du procédé conforme à l'Invention, les particules résultant de la micronisation du fénofibrate présentent une surface spécifique comprise entre 0,7 et 1,6 m²/g et, de préférence, voisine de 1 m²/g.

En effet, les Inventeurs ont constaté, sans toutefois pouvoir en expliquer les raisons, qu'en micronisant dans les mêmes conditions des fénofibrates issus de procédés de fabrication différents, on obtient des particules qui ne présentent pas la même surface spécifique d'un fénofibrate à l'autre et ce, à caractéristiques physiques identiques : densité, porosité (mesurée sur des pores de diamètre compris entre entre 1 et 100 nm), point de fusion, absence de variété polymorphe, système cristallin. Cette différence de surface spécifique intrinsèque est d'ailleurs corrélée à une énergie spécifique de transition différente, cependant en moindre proportion (écart de 10 à 20% entre les énergies de transition exprimées en J/g). Par ailleurs, les Inventeurs ont trouvé que, si la mouillabilité des particules de fénofibrate ou du mélange particules de fénofibrate/excipient(s) par le milieu liquide au cours de l'opération de granulation - dont dépend en grande partie la réussite de cette opération et, par conséquent, la qualité du granulat obtenu -, est améliorée par la présence d'un alcool dans ce milieu, elle l'est d'autant plus que les particules de fénofibrate présentent une surface spécifique comprise entre 0,7 et 1,6 m²/g et, plus particulièrement, proche de 1 m²/g.

Conformément à l'Invention, l'agent tensioactif est présent dans le milieu liquide utilisé pour la granulation à une concentration molaire qui est comprise entre 0,1 et 1 et, de préférence, entre 0,2 et 0,5, tandis que le rapport entre les volumes d'eau et d'alcool présents dans ce même milieu est, lui, compris entre 0,25 et 4 et, de préférence, entre 0,5 et 2. Ce rapport volumique eau/alcool sera avantageusement choisi en fonction de la surface spécifique des particules résultant de la micronisation du fénofibrate. En effet, il s'est avéré que, pour obtenir une granulation de même qualité, il est généralement nécessaire d'utiliser un rapport volumique eau/alcool d'autant plus élevé que la surface spécifique des particules de fénofibrate est elle-même plus élevée. A l'inverse, ce rapport nécessite généralement d'être abaissé lorsque la surface spécifique desdites particules est plus faible.

Selon encore une autre disposition avantageuse du procédé conforme à l'Invention, la quantité de milieu liquide utilisée pour la granulation est comprise entre 5 et 70% et, de préférence, entre 15 et 50% en poids rapporté au poids des particules de fénofibrate ou du mélange particules de fénofibrate/excipient(s) destinés à être soumis à cette granulation.

De manière préférée, l'agent tensioactif est du laurylsulfate de sodium, tandis que l'alcool hydromiscible est de l'éthanol. Toutefois, d'autres agents tensioactifs tels que le dioctylsulfosuccinate de sodium, le dodécylbenzènesulfonate de sodium, les halogénures de composés à base d'ammonium quaternaire, les polysorbates et les éthers d'alcools aliphatiques et de polyéthylène glycol, ainsi que d'autres alcools hydromiscibles comme l'isopropanol, sont également susceptibles d'être utilisés pour la mise en oeuvre du procédé conforme à l'Invention.

Selon une disposition particulièrement préférée du procédé conforme à l'Invention, celui-ci comprend :
(a) la micronisation du fénofibrate,
(b) le mélange du fénofibrate ainsi micronisé avec un ou plusieurs agents liants et/ou agents diluants et/ou agents désintégrants,
(c) la préparation du milieu liquide destiné à réaliser la granulation par dissolution de laurylsulfate de sodium dans une solution hydroalcoolique.
(d) la granulation du mélange obtenu à l'étape b) en présence dudit milieu liquide,
(e) le séchage du granulat ainsi obtenu,
(f) le calibrage de ce granulat, et
(g) le mélange dudit granulat avec un ou plusieurs agents lubrifiants et/ou agents d'écoulement et/ou agents colorants et/ou agents de sapidité.

Le procédé de préparation d'une formulation galénique objet de la présente Invention présente de nombreux avantages. En effet, outre de conduire à l'obtention de formulations galéniques conférant au fénofibrate une suprabiodisponibilité et autorisant, une diminution des doses de fénofibrate qui sont à ce jour préconisées pour l'obtention d'une efficacité thérapeutique comme il sera démontré ci-après, il présente l'avantage, de par le fait qu'il prévoit d'incorporer l'agent tensioactif directement dans le milieu liquide destiné à réaliser la granulation du fénofibrate, de comprendre une opération de moins que les procédés utilisés pour la préparation des médicaments à base de fénofibrate actuellement disponibles, et de permettre, ainsi, une meilleure reproductibilité des lots de fabrication et une économie substantielle sur le coût de production. En outre, il ne nécessite pas d'équipement spécifique et peut être aisément mis en oeuvre au moyen des appareillages dont sont classiquement équipés les laboratoires de galénique pour la fabrication de médicaments se présentant sous forme solide et, notamment, pour la granulation de principes actifs en milieu humide.

La présente Invention a, également, pour objet une formulation galénique comprenant du fénofibrate en tant que principe actif, caractérisée en ce qu'elle est susceptible d'être obtenue par la mise en oeuvre d'un procédé tel que précédemment défini.

La présente Invention a, encore, pour objet un médicament destiné à une administration par voie orale, caractérisé en ce qu'il comprend une quantité efficace d'une formulation galénique obtenue par la mise en oeuvre d'un procédé tel que précédemment défini.

Selon un mode de réalisation préféré de ce médicament, celui-ci se présente sous la forme de gélules, de préférence de gélatine. Il peut être, dans ces conditions, préparé par une opération consistant à remplir des gélules d'un calibre convenablement choisi avec une quantité prédéterminée d'une formulation galénique conforme à l'Invention.

En variante, il est toutefois possible de réaliser un tel médicament, sous la forme de comprimés nus ou pelliculés, par compression d'une formulation galénique conforme à l'Invention et, le cas échéant, pelliculage des produits résultant de ladite compression.

Selon un autre mode de réalisation préféré de ce médicament, il comprend une dose de fénofibrate comprise entre 100 et 200 mg par unité thérapeutique pour l'administration d'une unité thérapeutique par jour.

La présente Invention a, aussi, pour objet, l'utilisation d'un milieu liquide comprenant un agent tensioactif, de l'eau et un alcool hydromiscible pour réaliser la granulation de fénofibrate ou d'un mélange comprenant du fénofibrate et un ou plusieurs excipients.

La présente Invention a, en outre, pour objet l'utilisation de particules de fénofibrate présentant une surface spécifique comprise entre 0,7 et 1,6 m²/g et, de préférence, voisine de 1 m²/g pour la préparation d'un médicament destiné à une administration par voie orale.

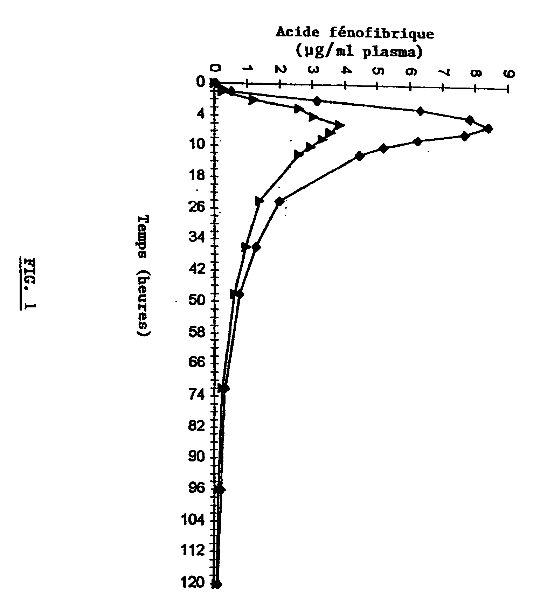

Outre les dispositions qui précédent, l'Invention comprend encore d'autres dispositions qui ressortiront du complément de description qui suit, qui se rapporte à des exemples de réalisation de médicaments conformes à l'Invention et de démonstration de leur pharmacocinétique, ainsi qu'à la Figure 1 annexée qui illustre les concentrations plasmatiques moyennes en acide fénofibrique, exprimées en µg par ml de plasma, obtenues chez des sujets sains respectivement après administration d'un médicament conforme à l'Invention (20 minutes après la prise d'un petit déjeuner standard) sous la forme d'une gélule dosée à 200 mg de fénofibrate (◆), et administration de 3 gélules de SECALIP® 100 (▲) dans les mêmes conditions.

### EXEMPLE 1 : Préparation d'un médicament se présentant sous la forme de gélules contenant 150 mg de fénofibrate

On prépare des gélules présentant chacune la composition qualitative et quantitative suivante :

| | |
|---|---|
| Fénofibrate | 150 mg |
| (surface spécifique après micronisation : 0,80-0,85 m²/g) | |
| Lactose monohydraté | 25,9 mg |
| Cellulose microcristalline | 13,5 mg |
| Povidone | 5,2 mg |
| Carboxyméthyl amidon sodique | 16,8 mg |
| Laurylsulfate de sodium | 4,5 mg |
| Stéarate de magnésium | 2,2 mg |

en opérant de la manière suivante :
- on micronise le fénofibrate dans un appareil à jet d'air de manière à obtenir une poudre dont les particules ont une dimension homogène de l'ordre de 5 à 10 µm et dont la surface spécifique, mesurée par la technique classique d'adsorption gazeuse en BET (*ADSORPTION SURFACE AREA AND POROSITY,* 1982, S.J. GREGG et K.S.W. SING, ACADEMIC PRESS), est comprise entre 0,80 et 0,85 m²/g ;
- on mélange cette poudre avec le lactose monohydraté, la povidone, la cellulose microcristalline et les 2/3 en poids du carboxyméthyl amidon sodique ;
- on réalise la granulation du mélange résultant en présence de 23% (en poids rapporté au poids de ce mélange) d'une solution hydroalcoolique (eau distillée/éthanol, 40/60, v/v) contenant 0,23 moles/litre de laurylsulfate de sodium ;
- on soumet les granules ainsi obtenus à un séchage pendant 10 heures à 55°C, puis à un calibrage de manière à ne retenir que ceux qui présentent une taille inférieure ou égale à 150 µm;
- on ajoute, à ces granules, le stéarate de magnésium et le reste du carboxyméthyl amidon sodique, et on mélange l'ensemble jusqu'à l'obtention d'une poudre homogène ; et
- on remplit des gélules de gélatine de taille n° 1 de manière à ce qu'elles renferment chacune 218,2 mg de cette poudre.

### EXEMPLE 2 : Préparation d'un médicament se présentant sous la forme de gélules contenant 200 mg de fénofibrate

On prépare des gélules présentant chacune la composition qualitative et quantitative suivante :

| | |
|---|---|
| Fénofibrate | 200 mg |
| (surface spécifique après micronisation: 1,38-1,50 m²/g) | |
| Lactose monohydraté | 50 mg |
| Povidone | 7 mg |
| Amidon prégélatinisé | 30 mg |
| Laurylsulfate de sodium | 6 mg |
| Stéarate de magnésium | 4,5 mg |
| Silice colloïdale anhydre | 1,5 mg |

en opérant de la manière suivante :
- on micronise le fénofibrate dans un appareil à jet d'air de manière à obtenir une poudre dont les particules ont une dimension homogène de l'ordre de 5 à 10 µm et dont la surface spécifique, mesurée par adsorption gazeuse en BET, est comprise entre 1,38 et 1,50 m²/g;
- on mélange cette poudre avec le lactose monohydraté, la povidone et l'amidon prégélatinisé ;
- on réalise la granulation du mélange ainsi obtenu en présence de 17% (en poids rapporté au poids de ce mélange) d'une solution hydroalcoolique (eau distillée/éthanol, 60/40, v/v) contenant 0,38 moles/litre de laurylsulfate de sodium ;
- on soumet les granules ainsi obtenus à un séchage et à un calibrage comme décrit dans l'exemple 1 ci-avant ;
- on ajoute, à ces granules, le stéarate de magnésium et la silice colloïdale anhydre, et on mélange l'ensemble jusqu'à l'obtention d'une poudre homogène ; puis
- on remplit des gélules de gélatine de taille n° 1 de manière à ce qu'elles renferment chacune 299 mg de cette poudre.

### EXEMPLE 3 : Etude pharmacocinétique d'un médicament conforme à l'Invention

L'aptitude d'une formulation galénique obtenue conformément à l'Invention à conférer au fénofibrate une *"suprabiodisponibilité"* a été établie par une étude visant à comparer le profil pharmacocinétique d'un médicament conforme à l'Invention - c'est-à-dire comprenant une telle formulation galénique - avec celui d'un médicament à base de fénofibrate de référence, en l'espèce le SECALIP® 100 qui, comme précédemment indiqué, remplace le LIPANTHYL® 100 sur le marché Français depuis 1991.

Pour ce faire, dans un essai croisé, 18 volontaires sains, d'âge compris entre 21 et 31 ans, ont reçu successivement, par voie orale, 1 gélule contenant 200 mg de fénofibrate et préparée conformément à l'exemple 2 - ci-après traitement A - et 3 gélules de SECALIP® 100 en une prise unique - ci-après traitement B -.

L'ordre des traitements a été randomisé et un intervalle de 14 jours a été laissé entre les deux traitements de manière à permettre une élimination totale de l'acide fénofibrique résultant du premier traitement.

Dans tous les cas, les traitements ont été administrés 20 minutes après la prise d'un petit déjeuner standard. Des prélèvements sanguins destinés à apprécier les taux plasmatiques de l'acide fénofibrique ont été effectués avant l'administration des traitements, puis 1, 2, 3, 4, 5, 6, 8, 10, 12, 24, 36, 48, 72, 96 et 120 heures après cette administration. Après centrifugation immédiate, le plasma a été séparé et conservé à -20°C jusqu'à la réalisation des dosages de l'acide fénofibrique, lesquels ont été effectués par chromatographie liquide à haute performance.

Pour tous les sujets, les paramètres pharmacocinétiques suivants ont été déterminés pour chacun des traitements :
- la concentration plasmatique maximale en acide fénofibrique observée au pic de concentration (Cₘₐₓ),
- le temps d'apparition de ce pic de concentration (Tₘₐₓ).
- l'aire sous la courbe des concentrations plasmatiques en acide fénofibrique en fonction du temps (AUC₀₋ₜ), et
- l'aire sous la courbe des concentrations plasmatiques en acide fénofibrique en fonction du temps extrapolée à l'infini (AUC_{0-α}).

Le Tableau 1 ci-dessous présente les moyennes ± écart-type des paramètres pharmacocinétiques ainsi obtenues.

**TABLEAU 1**

| | Traitement A | Traitement B |
|---|---|---|
| Cₘₐₓ (µg/ml) | 9,36 ± 3.41 | 3,80 ± 1,52 |
| Tₘₐₓ(h) | 4,4 ± 1,3 | 5,3 ± 2 |
| AUC₀₋ₜ (µg/ml.h) | 143 ± 73 | 84,2 ± 45,9 |
| AUC_{0-α} (µg/ml.h) | 148 ± 74 | 92,5 ± 46,2 |

La Figure 1 illustre, par ailleurs, les moyennes des concentrations plasmatiques en acide fénofibrique, exprimées en µg par ml de plasma, obtenues au cours des 120 heures suivant l'administration du traitement A (◆) et l'administration du traitement B (▲).

Les résultats présentés dans le Tableau 1 comme dans la Figure 1 montrent que, dans le cas du traitement A :
- d'une part, la quantité de fénofibrate résorbée est bien supérieure puisque, non seulement la concentration plasmatique maximale en acide fénofibrique au pic de concentration (Cₘₐₓ) est en moyenne plus de 2 fois supérieure (2,4 fois au vu du Tableau 1 et 2,16 fois au vu de la Figure 1) à celle obtenue avec le traitement B, mais de plus les valeurs moyennes des aires sous la courbe des concentrations plasmatiques en acide fénofibrique en fonction du temps (AUC₀₋ₜ et AUC_{0-α}) sont significativement plus élevées que celles enregistrées avec le traitement B, et
- d'autre part, le fénofibrate est résorbé aussi rapidement puisque, selon le Tableau 1, le pic de concentration se situe en moyenne une heure avant l'apparition du pic de concentration généré par le traitement B, tandis que sur la Figure 1, ces deux pics coïncident dans le temps, et ce, bien que la dose de fénofibrate administrée soit plus faible que celle ingérée dans le cas du traitement B (200 mg au lieu de 3x100 mg).

Par ailleurs, dans la mesure où il a été montré dans la littérature qu'il existe une bioéquivalence cinétique entre 3 gélules de SECALIP® 100 - puisque cette spécialité pharmaceutique a remplacé, à l'identique, le LIPANTHYL® 100 - et 1 gélule de LIPANTHYL® 200 Micronisé, les résultats présentés dans le Tableau 1 et la Figure 1 signifient qu'une formulation galénique préparée conformément à l'Invention et comprenant 200 mg de fénofibrate est à même d'assurer une résorption intestinale de ce principe actif plus complète et plus rapide qu'une gélule de LIPANTHYL® 200 Micronisé - qui est le médicament à base de fénofibrate présentant à ce jour le meilleur rapport effet/dose - et qu'elle autorise l'administration de doses quotidiennes de fénofibrate inférieures à 200 mg pour l'obtention d'un même bénéfice thérapeutique.

Une telle formulation galénique rend ainsi possible la préparation de médicaments propres à diminuer les risques d'effets secondaires et à améliorer le confort des patients et l'observance d'un traitement par le fénofibrate au long cours.

## Revendications

1. Procédé de préparation d'une formulation galénique comprenant du fénofibrate en tant que principe actif, **caractérisé en ce qu'**il comprend les étapes suivantes :
(a) la micronisation du fénofibrate,
(b) la granulation du fénofibrate en présence d'un milieu liquide comprenant un agent tensioactif, de l'eau et un alcool hydromiscible, et
(c) le séchage du granulat ainsi obtenu.

2. Procédé selon la revendication 1, **caractérisé en ce qu'**il comprend, préalablement et/ou postérieurement à la granulation, l'addition d'un ou plusieurs excipients choisis parmi les agents liants, les agents diluants, les agents désintégrants, les agents lubrifiants, les agents d'écoulement, les colorants et les agents de sapidité.

3. Procédé selon la revendication 1 ou la revendication 2, **caractérisé en ce qu'**il comprend, postérieurement au séchage du granulat et antérieurement au mélange éventuel de ce dernier avec un ou plusieurs excipients, le calibrage de ce granulat.

4. Procédé selon l'une quelconque des revendications 1 à 3, **caractérisé en ce que** la micronisation du fénofibrate est réalisée de manière à obtenir une poudre dont les particules ont une dimension homogène égale au plus à 10 µm et, de préférence, comprise entre 5 et 10 µm.

5. Procédé selon l'une quelconque des revendications 1 à 4, **caractérisé en ce que** les particules résultant de la micronisation du fénofibrate présentent une surface spécifique comprise entre 0,7 et 1,6 m²/g et, de préférence, voisine de 1 m²/g.

6. Procédé selon l'une quelconque des revendications 1 à 5, **caractérisé en ce que** l'agent tensioactif est présent dans le milieu liquide utilisé pour la granulation à une concentration molaire qui est comprise entre 0,1 et 1 et, de préférence, entre 0,2 et 5.

7. Procédé selon l'une quelconque des revendications 1 à 6, **caractérisé en ce que** le rapport entre les volumes d'eau et d'alcool présents dans le milieu liquide utilisé pour la granulation est compris entre 0,25 et 4 et, de préférence, entre 0,5 et 2.

8. Procédé selon l'une quelconque des revendications 1 à 7, **caractérisé en ce que** la quantité de milieu liquide utilisée pour la granulation est comprise entre 5 et 70% et, de préférence, entre 15 et 50% en poids rapporté au poids des particules de fénofibrate ou du mélange particules de fénofibrate/excipient(s) destinés à être soumis à cette granulation.

9. Procédé selon l'une quelconque des revendications 1 à 8, **caractérisé en ce que** l'agent tensioactif est du laurylsulfate de sodium.

10. Procédé selon l'une quelconque des revendications 1 à 9, **caractérisé en ce que** l'alcool hydromiscible est de l'éthanol.

11. Procédé selon l'une quelconque des revendications 1 à 10, **caractérisé en ce qu'**il comprend :
(a) la micronisation du fénofibrate,
(b) le mélange du fénofibrate ainsi micronisé avec un ou plusieurs agents liants et/ou agents diluants et/ou agents désintégrants,
(c) la préparation du milieu liquide destiné à réaliser la granulation par dissolution de laurylsulfate de sodium dans une solution hydroalcoolique,
(d) la granulation du mélange obtenu à l'étape b) en présence dudit milieu liquide,
(e) le séchage du granulat ainsi obtenu,
(f) le calibrage de ce granulat, et
(g) le mélange dudit granulat avec un ou plusieurs agents lubrifiants et/ou agents d'écoulement et/ou agents colorants et/ou agents de sapidité.

12. Formulation galénique comprenant du fénofibrate en tant que principe actif, **caractérisée en ce qu'**elle est susceptible d'être obtenue par la mise en oeuvre d'un procédé selon l'une quelconque des revendications 1 à 11.

13. Médicament destiné à une administration par voie orale, **caractérisé en ce qu'**il comprend une quantité efficace d'une formulation galénique obtenue par la misé en oeuvre d'un procédé selon l'une quelconque des revendications 1 à 11.

14. Médicament selon la revendication 13, **caractérisé en ce qu'**il se présente sous la forme de gélules, de préférence de gélatine.

15. Médicament selon la revendication 13 ou la revendication 14, **caractérisé en ce qu'**il comprend une dose de fénofibrate comprise entre 100 et 200 mg par unité thérapeutique pour l'administration d'une unité thérapeutique par jour.

16. Utilisation d'un milieu liquide comprenant un agent tensioactif, de l'eau et un alcool hydromiscible pour réaliser la granulation de fénofibrate ou d'un mélange comprenant du fénofibrate et un ou plusieurs excipients.

17. Utilisation de particules de fénofibrate présentant une surface spécifique comprise entre 0,7 et 1,6 m²/g et, de préférence, voisine de 1 m²/g pour la préparation d'un médicament destiné à une administration par voie orale.

## Claims

1. Process for preparing a galenic formulation containing fenofibrate as active ingredient, **characterised in that** it comprises the following steps:
(a) micronising the fenofibrate,
(b) granulating the fenofibrate in the presence of a liquid medium containing a surfactant, water and a water-miscible alcohol, and
(c) drying the granules thus obtained.

2. Process according to claim 1, **characterised in that** it comprises, prior to and/or subsequent to the granulation, the addition of one or more excipients selected from among binders, diluents, disintegrants, lubricants, flow agents, colourings and flavourings.

3. Process according to claim 1 or 2, **characterised in that** it comprises, after the drying of the granules and prior to any mixing thereof with one or more excipients, the screening of the granules.

4. Process according to any one of claims 1 to 3, **characterised in that** the micronisation of the fenofibrate is carried cut so as to obtain a powder the particles of which have a uniform size of not more than 10 µm and preferably between 5 and 10 µm.

5. Process according to any one of claims 1 to 4, **characterised in that** the particles resulting from the micronisation of the fenofibrate have a specific surface area of between 0.7 and 1.6 m²/g and preferably approximately 1 m²/g.

6. Process according to any one of claims 1 to 5, **characterised in that** the surfactant is present in the liquid medium used for the granulation in a molar concentration which is between 0.1 and 1 and preferably between 0.2 and 5.

7. Process according to any one of claims 1 to 6, **characterised in that** the ratio between the volumes of water and alcohol present in the liquid medium used for the granulation is between 0.25 and 4 and preferably between 0.5 and 2.

8. Process according to any one of claims 1 to 7, **characterised in that** the quantity of liquid medium used for the granulation is between 5 and 70% and preferably between 15 and 50% by weight, based on the weight of the particles of fenofibrate or the mixture of particles of fenofibrate/excipient(s) intended to be subjected to this granulation.

9. Process according to any one of claims 1 to 8, **characterised in that** the surfactant is sodium laurylsulphate.

10. Process according to any one of claims 1 to 9, **characterised in that** the water-miscible alcohol is ethanol.

11. Process according to any one of claims 1 to 10, **characterised in that** it comprises:
(a) micronising the fenofibrate,
(b) mixing the fenofibrate thus micronised with one or more binders and/or diluents and/or disintegrants,
(c) preparing the liquid medium intended for the granulation by dissolving sodium laurylsulphate in an aqueous alcoholic solution,
(d) granulating the mixture obtained in step b) in the presence of said liquid medium,
(e) drying the granules thus obtained,
(f) screening the granules, and
(g) mixing said granules with one or more lubricants and/or flow agents and/or colourings and/or flavourings.

12. Galenic formulation comprising fenofibrate as active ingredient, **characterised in that** it may be obtained by carrying out a process according to any one of claims 1 to 11.

13. Medicament intended for oral administration, **characterised in that** it comprises an effective amount of a galenic formulation obtained by carrying out a process according to any one of claims 1 to 11.

14. Medicament according to claim 13, **characterised in that** it is in the form of capsules, preferably gelatine capsules.

15. Medicament according to claim 13 or claim 14, **characterised in that** it comprises a dose of fenofibrate of between 100 and 200 mg per dosage unit for administration of one dosage unit per day.

16. Use of a liquid medium containing a surfactant, water and a water-miscible alcohol for granulating fenofibrate or a mixture containing fenofibrate and one or more excipients.

17. Use of fenofibrate particles having a specific surface area of between 0.7 and 1.6 m²/g and preferably approximately 1 m²/g for the preparation of a medicament intended for oral administration.

## Patentansprüche

1. Verfahren zur Herstellung einer galenischen Formulierung, die Fenofibrat als Wirkstoff enthält, **dadurch gekennzeichnet, dass** es die folgenden Stufen umfasst:
(a) Mikronisierung des Fenofibrats,
(b) Granulierung des Fenofibrats in Gegenwart eines flüssigen Mediums, das ein grenzflächenaktives Mittel, Wasser und einen mit Wasser mischbaren Alkohol umfasst, und
(c) Trocknung des so erhaltenen Granulats.

2. Verfahren nach Anspruch 1, **dadurch gekennzeichnet, dass** es vor und/oder nach der Granulierung das Zugeben eines Exzipiens oder mehrerer Exzipientien, ausgewählt aus Bindemitteln, Verdünnungsmitteln, Trennmitteln, Gleitmitteln, thixotropen Mitteln, Färbemitteln und Geschmacksmitteln, umfasst.

3. Verfahren nach Anspruch 1 oder Anspruch 2, **dadurch gekennzeichnet, dass** es vor der Trocknung des Granulats und nach dem eventuellem Vermischen des letztgenannten mit einem Exzipiens oder mehreren Exzipientien, die Klassierung des Granulats umfasst.

4. Verfahren nach einem der Ansprüche 1 bis 3, **dadurch gekennzeichnet, dass** die Mikronisierung des Fenofibrats so durchgeführt wird, dass ein Pulver erhalten wird, dessen Partikel eine homogene Abmessung haben, welche höchstens 10 µm und vorzugsweise 5 bis 10 µm ist.

5. Verfahren nach einem der Ansprüche 1 bis 4, **dadurch gekennzeichnet, dass** die aus der Mikronisierung des Fenofibrats resultierenden Partikel eine spezifische Oberfläche von 0,7 bis 1,6 m²/g und vorzugsweise von etwa 1 m²/g aufweisen.

6. Verfahren nach einem der Ansprüche 1 bis 5, **dadurch gekennzeichnet, dass** das grenzflächenaktive Mittel in dem flüssigen Medium, das zur Granulierung verwendet wird, in einer molaren Konzentration vorliegt, die zwischen 0,1 und 1 und vorzugsweise zwischen 0,2 und 5 liegt.

7. Verfahren nach einem der Ansprüche 1 bis 6, **dadurch gekennzeichnet, dass** das Verhältnis zwischen den Volumina an Wasser und Alkohol in dem flüssigen Medium, das zur Granulierung verwendet wird, zwischen 0,25 und 4 und vorzugsweise zwischen 0,5 und 2 liegt.

8. Verfahren nach einem der Ansprüche 1 bis 7, **dadurch gekennzeichnet, dass** die Menge an flüssigem Medium, die zur Granulierung verwendet wird, zwischen 5 und 70 Gew.-% und vorzugsweise zwischen 15 und 50 Gew.-%, bezogen auf das Gewicht der Fenofibratpartikel oder der Partikel des Gemisches aus Fenofibrat/Exzipiens (Exzipientien), die dieser Granulierung unterzogen werden sollen, beträgt.

9. Verfahren nach einem der Ansprüche 1 bis 8, **dadurch gekennzeichnet, dass** das grenzflächenaktive Mittel Natriumlaurylsulfat ist.

10. Verfahren nach einem der Ansprüche 1 bis 9, **dadurch gekennzeichnet, dass** der mit Wasser mischbare Alkohol Ethanol ist.

11. Verfahren nach einem der Ansprüche 1 bis 10, **dadurch gekennzeichnet, dass** es umfasst:
(a) Mikronisierung von Fenofibrat,
(b) Vermischen des so mikronisierten Fenofibrats mit einem oder mehreren Bindemittel(n) und/oder Verdünnungsmittel(n) und/oder Trennmittel(n),
(c) Herstellung des flüssigen Mediums, das zur Durchführung der Granulierung bestimmt ist, durch Auflösen von Natriumlaurylsulfat in einer wässrig-alkoholischen Lösung,
(d) Granulierung des in Stufe (b) erhaltenen Gemisches in Gegenwart des flüssigen Mediums,
(e) Trocknung des so erhaltenen Granulats,
(f) Klassierung dieses Granulats und
(g) Mischen des Granulats mit einem oder mehreren Gleitmitteln und/oder thixotropen Mitteln und/oder Färbemitteln und/oder Geschmacksmitteln.

12. Galenische Formulierung, die Fenofibrat als Wirkstoff enthält, **dadurch gekennzeichnet ist, dass** sie durch Durchführung eines Verfahrens nach einem der Ansprüche 1 bis 11 erhalten werden kann.

13. Arzneimittel, dass zur Verabreichung auf oralem Weg bestimmt ist, **dadurch gekennzeichnet**, das es eine wirksame menge der galenischen Formulierung umfasst, die durch Durchführung eines Verfahrens nach einem der Ansprüche 1 bis 11 erhalten wird.

14. Arzneimittel nach Anspruch 13, **dadurch gekennzeichnet, dass** es in Form von Kapseln, vorzugsweise Gelatinekapseln, vorliegt.

15. Arzneimittel nach Anspruch 13 oder Anspruch 14, **dadurch gekennzeichnet, dass** es eine Dosis an Fenofibrat von 100 bis 200 mg pro therapeutischer Einheit zur Verabreichung einer therapeutischen Einheit pro Tag umfasst.

16. Verwendung eines flüssigen Mediums, dass ein grenzflächenaktives Mittel, Wasser und einen mit Wasser mischbaren Alkohol umfasst, zur Durchführung der Granulierung von Fenofibrat oder einem Gemisch, dass Fenofibrat und ein Exzipiens oder mehrere Exzipientien umfasst.

17. Verwendung von Fenofibratpartikeln, die eine spezifische Oberfläche aufweisen, die 0,7 bis 1,6 m²/g und vorzugsweise etwa 1 m²/g ist, zur Herstellung eines Arzneimittels, dass zur Verabreichung auf oralem Weg bestimmt ist.
